# EUROPEAN PATENT SPECIFICATION

(11) **EP 3 861 936 B1**
(45) Date of publication and mention of the grant of the patent: **03.04.2024**
(21) Application number: 21155692.3
(22) Date of filing: 08.02.2021
(51) Int. Cl.: A61B 8/08, A61B 8/00, A61B 8/12

(54) **ULTRASOUND DIAGNOSTIC APPARATUS AND METHOD OF PROCESSING ULTRASOUND IMAGE**
ULTRASCHALLDIAGNOSEVORRICHTUNG UND VERFAHREN ZUR VERARBEITUNG VON ULTRASCHALLBILDERN
APPAREIL DE DIAGNOSTIC ULTRASONIQUE ET PROCÉDÉ DE TRAITEMENT D'UNE IMAGE ULTRASON

(30) Priority: 10.02.2020 KR 20200015626
(43) Date of publication of application: 11.08.2021
(73) Proprietor: Samsung Medison Co., Ltd., Hongcheon-gun, Gangwon-do 25108 (KR)
(72) Inventor: LEE, Dongeun, 05340 SEOUL (KR); LEE, Jin-Yong, 05340 SEOUL (KR)
(74) Representative: Arnold & Siedsma

(56) References cited:
- US-A1- 2008 167 553
- US-A1- 2016 151 041
- US-A1- 2019 008 674
- DZIADOSZ MARGARET ET AL: "Uterocervical angle: a novel ultrasound screening tool to predict spontaneous preterm birth", AMERICAN JOURNAL OF OBSTETRICS & GYNECOLOGY, MOSBY, ST LOUIS, MO, US, vol. 215, no. 3, 24 March 2016 (2016-03-24), XP029702096, ISSN: 0002-9378, DOI: 10.1016/J.AJOG.2016.03.033
- LEITICH ET AL: "Cervical length and dilatation of the internal cervical os detected by vaginal ultrasonography as markers for preterm delivery: A systematic review", AMERICAN JOURNAL OF OBSTETRICS & GYNECOLOGY, MOSBY, ST LOUIS, MO, US, vol. 181, no. 6, 1 December 1999 (1999-12-01), pages 1465-1472, XP005691827, ISSN: 0002-9378, DOI: 10.1016/S0002-9378(99)70407-2
- OWEN ET AL: "What we have learned about cervical ultrasound", SEMINARS IN PERINATOLOGY, W.B. SAUNDERS, GB, vol. 27, no. 3, 1 June 2003 (2003-06-01), pages 194-203, XP004666079, ISSN: 0146-0005, DOI: 10.1016/S0146-0005(03)00021-1
- Sabiani Laura ET AL: "Cervical HI-RTE elastography and pregnancy outcome: a prospective study", European Journal of Obstetrics & Gynecology and Reproductive Biology, vol. 186, 1 March 2015 (2015-03-01), pages 80-84, XP093099500, IE ISSN: 0301-2115, DOI: 10.1016/j.ejogrb.2015.01.016

## Description

### BACKGROUND

### 1. Field

The disclosure relates to an ultrasound diagnostic apparatus receiving an ultrasound image from a probe and a method of controlling the same.

### 2. Description of Related Art

An ultrasound diagnostic apparatus irradiates an ultrasound signal generated from a transducer of a probe to an object, receives information of a signal reflected from the object, and obtains at least one image of a site (e.g., soft tissue or blood flow) inside the object.

The ultrasound diagnostic apparatus has advantages in that it is compact and inexpensive, and has non-invasive and non-destructive characteristics compared to other imaging devices such as X-ray devices, computerized tomography (CT) scanners, magnetic resonance image (MRI), nuclear medicine diagnostic devices, etc., and thus is widely used for cardiac, breast, abdominal, urinary, and obstetrical diagnoses.

The probe including the transducer for irradiating the ultrasound signal to the object and receiving an ultrasound echo signal reflected from the object may be connected to the ultrasound diagnostic apparatus. The ultrasound diagnostic apparatus may include a transceiver (hereinafter referred to as a channel) that transmits and receives signals to and from the probe. Conventional general ultrasound diagnostic apparatus uses a switch when the number of channels is less than the number of elements of the transducer. That is, through a switching operation, the ultrasound diagnostic apparatus selectively connects signals received by the elements of a plurality of transducers.

Conventionally, in order to determine whether childbirth is possible, a method of determining a degree of opening of a cervix by inserting a finger into a mother's vagina was used by a doctor.

However, the doctor's seismic examination method can make an inaccurate diagnosis because the doctor's finger thickness is not constant, and causes pain and discomfort to the mother.

Documents US2019/008674, US2016/151041, US2008/167553 disclose ultrasound apparatuses and methods controlling said apparatuses, according to the preamble of the appended independent claims.

### Brief Summary of the Invention

The invention is defined by the appended claims.

### Outline of the disclosure

An aspect of the disclosure provides an ultrasound diagnostic apparatus capable of accurately diagnosing a childbirth state and whether or not a doctor's seismic examination is required.

Additional aspects of the disclosure will be set forth in part in the description which follows and, in part, will be obvious from the description, or may be learned by practice of the disclosure.

According to an aspect of the disclosure, there is provided an ultrasound diagnostic apparatus including: a display; a probe configured to obtain image data of a cervix using ultrasound; and a controller configured to determine a degree of expansion of the cervix based on the image data, to generate an ultrasound image of the cervix, and to control the display so the degree of expansion of the cervix is schematically displayed on the ultrasound image.

The controller may be configured to schematically display at least one of a cervical angle or the degree of expansion of the cervix at a position corresponding to the cervix in the ultrasound image.

The controller may be configured to calculate a score of the cervical angle based on a first predetermined criterion, and to determine a childbirth stage based on the calculated score of the cervical angle.

The controller may be configured to score an expansion of the cervix based on a preset second criterion and determine a childbirth stage.

The controller may be configured to calculate a childbirth time based on the degree of expansion of the cervix.

The controller may be configured to determine whether a fetus is prematurely born based on the image data, and to control the display to display the determination result on the display.

The controller may be configured to measure an anterior cervical angle or a posterior cervical angle, and to determine that it is an abnormal childbirth in response to the anterior cervical angle exceeding a preset first angle or the posterior cervical angle being less than a preset second angle.

In response to the anterior cervical angle being more than 101° or less than 140°, or the posterior cervical angle being more than 101° or less than 140°, the controller may be configured to determine that it is the abnormal childbirth.

The ultrasound diagnostic apparatus may further include a storage configured to store the image data.

According to another aspect of the disclosure, there is provided a method of controlling an ultrasound diagnostic apparatus including: obtaining, by a probe, image data of a cervix using ultrasound; determining, by a controller, a degree of expansion of the cervix based on the image data; generating, by the controller, an ultrasound image of the cervix; and controlling, by the controller, a display so that the degree of expansion of the cervix is schematically displayed on the ultrasound image.

The controlling may further include schematically displaying at least one of a cervical angle or the degree of expansion of the cervix at a position corresponding to the cervix in the ultrasound image.

The controlling may further include calculating a score of the cervical angle based on a first predetermined criterion; and determining a childbirth stage based on the calculated score of the cervical angle.

The controlling may further include scoring an expansion of the cervix based on a preset second criterion and determine a childbirth stage.

The controlling may further include calculating a childbirth time based on the degree of expansion of the cervix.

The controlling may further include determining whether a fetus is prematurely born based on the image data; and controlling the display to display the determination result on the display.

The controlling may further include measuring an anterior cervical angle or a posterior cervical angle; and determining that it is an abnormal childbirth in response to the anterior cervical angle exceeding a preset first angle or the posterior cervical angle being less than a preset second angle.

The controlling may further include, in response to the anterior cervical angle being more than 101° or less than 140°, or the posterior cervical angle being more than 101° or less than 140°, determining that it is the abnormal childbirth.

The method may further include storing, by a storage, the image data.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is an external perspective view of an ultrasound diagnostic apparatus according to an embodiment.
FIG. 2 is an external view of a probe including a one-dimensional array transducer element.
FIG. 3 is an external view of a probe including a two-dimensional array transducer element.
FIG. 4 is a control block diagram of an ultrasound diagnostic apparatus according to an embodiment.
FIG. 5 is a view illustrating a process of obtaining image data of a cervix of an object according to an embodiment.
FIG. 6 is a view illustrating a cervical angle appearing on a display according to an embodiment.
FIG. 7 is a view illustrating a degree of expansion of a cervix appearing on a display according to an embodiment.
FIG. 8 is a view illustrating a process of determining whether a fetus is prematurely born by a controller according to an embodiment.
FIG. 9 is a view illustrating how a controller determines a mother's childbirth state based on a cervical angle, according to an embodiment.
FIG. 10 is a view illustrating a process in which a controller determines a childbirth time and a risk of premature birth based on a degree of expansion of a cervix, according to an embodiment.

### DETAILED DESCRIPTION

Like reference numerals refer to like elements throughout the specification. Not all elements of the embodiments of the disclosure will be described, and the description of what are commonly known in the art or what overlap each other in the exemplary embodiments will be omitted. The terms as used throughout the specification, such as "- part," "- module," "- member," "- block," etc., may be implemented in software and/or hardware, and a plurality of "- parts," "- modules," "- members," or "~ blocks" may be implemented in a single element, or a single "-part," "- module," "- member," or "~ block" may include a plurality of elements.

It will be further understood that the term "connect" and its derivatives refer both to direct and indirect connection, and the indirect connection includes a connection over a wireless communication network.

The terms "include (or including)" and "comprise (or comprising)" are inclusive or open-ended and do not exclude additional, unrecited elements or method steps, unless otherwise mentioned. It will be further understood that the term "member" and its derivatives refer both to when a member is in contact with another member and when another member exists between the two members.

Further, when it is stated that a layer is "on" another layer or substrate, the layer may be directly on another layer or substrate or a third layer may be disposed therebetween.

It will be understood that, although the terms first, second, third, etc., may be used herein to describe various elements, components, regions, layers and/or sections, these elements, components, regions, layers and/or sections should not be limited by these terms. These terms are only used to distinguish one element, component, region, layer or section from another region, layer or section.

It is to be understood that the singular forms "a," "an," and "the" include plural references unless the context clearly dictates otherwise.

Reference numerals used for method steps are merely used for convenience of explanation, but not to limit an order of the steps. Thus, unless the context clearly dictates otherwise, the written order may be practiced otherwise.

Hereinafter, operation principles and embodiments of the disclosure will be described with reference to accompanying drawings.

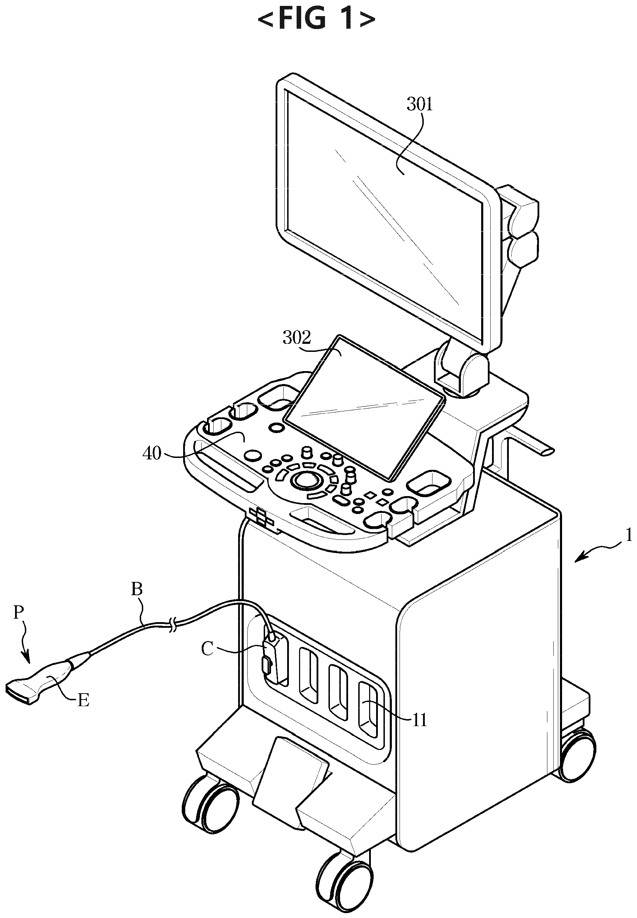

FIG. 1 is an external perspective view of an ultrasound diagnostic apparatus according to an embodiment.

Referring to FIG. 1, an ultrasound diagnostic apparatus 1 may be connected to a probe 100 that transmits an ultrasound signal to an object, receives an echo ultrasound signal from the object, and converts it into an electrical signal.

The ultrasound diagnostic apparatus 1 may be connected to the probe 100 through a wired communication network or a wireless communication network. The ultrasound diagnostic apparatus 1 may be a work station with a display 30 and an input device 40. In addition, the ultrasound diagnostic apparatus 1 may exchange various information with an external device through the wired communication network or the wireless communication network.

The input device 40 may receive various control commands, such as an operation for the connected probe 100 and an operation command of the ultrasound diagnostic apparatus 1 that generates an ultrasound image. The input device 40 may be implemented with various hardware devices such as a keyboard, a foot switch, or a foot pedal method. For example, when the input device 40 is implemented as a keyboard, the keyboard may include at least one of a switch, a key, a joystick, and a trackball. As another example, the keyboard may be implemented in software such as a graphic user interface. In this case, the keyboard may be displayed through the second display 302. A foot switch or foot pedal may be provided under the ultrasound diagnostic apparatus 1, and the user may control the operation of the ultrasound diagnostic apparatus 1 by using the foot pedal.

The display 300 may display an ultrasound image generated by the ultrasound diagnostic apparatus 1 and various graphic user interfaces.

The display 300 according to an example may include a first display 301 and a second display 302.

The ultrasound image displayed on the first display 301 may be a two-dimensional ultrasound image or a three-dimensional ultrasound image, and various ultrasound images may be displayed according to an operation mode of the ultrasound diagnostic apparatus 1. In addition, the first display 301 may display not only menus or guides necessary for ultrasound diagnosis, but also information on the operation state of the probe 100.

The second display 302 may provide related information such as a menu for optimizing an ultrasound image or an auxiliary image, or may provide a graphic user interface to a user. When the second display 302 serves as the input device 40, a graphic user interface having the same shape as a button included in the input device 40 may be displayed on the second display 302.

On the other hand, the form of the ultrasound diagnostic apparatus 1 is not necessarily limited to that illustrated in FIG. 1. For example, the ultrasound diagnostic apparatus 1 may be implemented in the form of a smartphone, as well as a laptop, a desktop, and a tablet PC. In addition, the ultrasound diagnostic apparatus 1 may be implemented in the form of a mobile terminal such as a personal digital assistant (PDA), a watch that can be attached to a user's body, and a wearable terminal in the form of glasses.

The object may be a living body of a human or animal, or an in vivo tissue such as blood vessels, bones, muscles, etc., but is not limited thereto, and may be the object as long as its internal structure can be imaged by the ultrasound diagnostic apparatus 1.

The probe 100 may include a transducer E that is provided in the housing to irradiate ultrasound to an object, receives echo ultrasound reflected from the object, and converts electrical pulse signal and ultrasound to each other, a male connector C that is physically coupled with a female connector 11 of the ultrasound diagnostic apparatus 1 to transmit and receive signals to the ultrasound diagnostic apparatus 1, and a cable B connecting the ultrasound diagnostic apparatus 1 and the probe 100.

The transducer E can generate ultrasound according to the applied AC power. Particularly, the transducer E may receive AC power from a power storage device, for example, a battery, inside the probe 100. A vibrator (hereinafter referred to as a transducer element) of the transducer E may generate ultrasound by vibrating according to the supplied AC power.

In addition, the transducer E receives the signal reflected by the object, that is, echo ultrasound. The transducer E converts the echo ultrasound into an electrical signal. The echo ultrasound has various frequency bands or energy intensity to generate various ultrasound images according to the diagnosis mode.

The probe 100 transmits the analog signal or digital signal converted from ultrasound by each element of the transducer E to the ultrasound diagnostic apparatus 1 through the cable B. On the other hand, the disclosed probe 100 is not necessarily connected to the ultrasound diagnostic apparatus 1 through the cable B. The probe 100 and the ultrasound diagnostic apparatus 1 may transmit and receive signals through wired or wireless communication.

The ultrasound diagnostic apparatus 1 may include a probe select assembly (PSA) board that receives signals transmitted by the probe 100. The PSA board may include a plurality of female connectors 11 so that a plurality of probes 100 can be connected, and may include a switch 10 and a plurality of channels 20 provided inside the PSA board. The PSA board transmits the received signal to a beamformer 30. A detailed description of the ultrasound diagnostic apparatus 1 will be described later through other drawings.

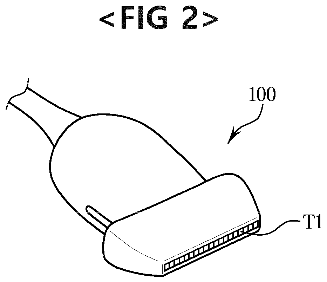

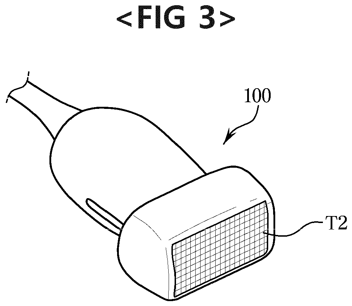

FIG. 2 is an external view of a probe including a one-dimensional array transducer element, and FIG. 3 is an external view of a probe including a two-dimensional array transducer element. It will be described together below in order to avoid redundant description.

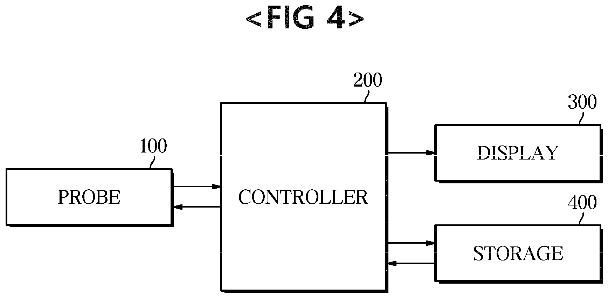

FIG. 4 is a control block diagram of an ultrasound diagnostic apparatus according to an embodiment.

Referring to FIG. 4, the ultrasound diagnostic apparatus 1 may include the probe 100, a controller 200, the display 300, or a storage 400.

Referring to FIGS. 2, 3, and 4, the probe 100 the probe 100 may convert the pulse signal received from the main body into an ultrasound signal, transmit the ultrasound signal to a specific site inside the object, and receive the echo ultrasound signal reflected from the specific site inside the object, convert it back into the pulse signal, and transmit it to the ultrasound diagnostic apparatus 1.

Here, the object may be a human uterus or cervix, but is not limited thereto.

Particularly, referring to FIGS. 2 and 3, the probe 100 may include a transducer element that converts an electrical pulse signal and an ultrasound signal to each other in order to transmit an ultrasound signal into the object. The transducer array is composed of a single or a plurality of transducer elements.

The transducer array may be a one-dimensional array or a two-dimensional array. In an embodiment, the transducer E may include a one-dimensional transducer array as illustrated in FIG. 2.

Each of the transducer elements constituting the one-dimensional transducer array may convert the ultrasonic signal into an electrical signal and vice versa. For this, the transducer element may be a magnetostrictive ultrasonic transducer using the magnetostrictive effect of a magnetic material, a piezoelectric ultrasonic transducer or a piezoelectric Micromachined Ultrasonic Transducer (pMUT) using the piezoelectric effect of a piezoelectric material, or a capacitive Micromachined Ultrasonic Transducer (cMUT) that transmits and receives ultrasonic waves using vibration of several hundreds or thousands of micromachined thin films.

Meanwhile, the one-dimensional transducer array may be linearly aligned or may be convexly aligned as illustrated in FIG. 2. In both cases, the probe 100 may operate according to the same operation principle, however, when the probe 100 includes the convex transducer E in a convex shape, the ultrasound waves irradiated from the transducer E may be in the shape of a fan, and accordingly, the ultrasound image may also be created in the shape of a fan.

As another example, the transducer E may include a two-dimensional transducer array as illustrated in FIG. 3. In the case of including a 2D transducer array, the interior of the object can be imaged in 3D. In addition, even if the transducer array of the probe 100 is arranged in one dimension, the probe 100 may mechanically move the 1D transducer array and transmit an echo ultrasound signal capable of generating a 3D ultrasound image to the ultrasound diagnostic apparatus 1 by obtaining volume information inside the object.

Each transducer element constituting the 2D transducer array may be the same as the transducer element constituting the 1D transducer array.

Meanwhile, the 2D transducer array may include a larger number of transducer elements than the 1D transducer array. The ultrasound diagnostic apparatus 1 may include the plurality of channels 20 to receive signals transmitted by a large number of transducer elements.

Conventionally, when the number of channels is less than the transducer element of the connected probe 100, the conventional general ultrasound diagnostic apparatus processes the received signal by using the switch 10. However, the disclosed ultrasound diagnostic apparatus 1 may perform signal processing through the switch 10 even when the number of channels is greater than the transducer element of the probe 100 to which it is connected.

The controller 200 may determine an cervical angle based on the image data obtained by the probe 100, generate an ultrasound image of the cervix based on the image data obtained by the probe 100, and control the display 300 so that the determined cervical angle is schematically displayed on the generated ultrasound image.

Here, the schematic angle may represent an angle at an intersection of two straight lines and two straight lines. In addition, the cervical angle may include the cervical angle of an anterior and a posterior, and a position of the cervix may change from the anterior to the posterior as childbirth progresses.

In addition, the controller 200 may control the display 300 to display the schematic angle at the position corresponding to the cervix.

In addition, the controller 200 may determine a degree of expansion of the cervix based on the data obtained by the probe 100.

The controller 200 may score the cervical angle based on a first preset criterion, and score the degree of expansion of the cervix based on a second preset criterion.

**<Table 1 >**

| | score | | | |
|---|---|---|---|---|
| | 0 | 1 | 2 | 3 |
| cervical position | posterior | middle | anterior | - |
| cervical elasticity | rigidity | middle | softness | - |
| narrowing of cervix | 0∼30% | 40∼50% | 60∼70% | 80% or more |
| cervical expansion | not open | 1∼2cm | 3∼4cm | 5cm or more |
| degree of expansion of cervical angle | -3 | -2 | -1/0 | 1/2 |
| cervical angle | posterior angle less than 100°, anterior angle greater than | anterior and posterior angle greater than 101° less than | posterior angle greater than 141°, anterior angle less than | |
| | 141° | 141° | 100° | |

Here, the preset first criterion may be a cervical angle item illustrated in Table 1 above. Also, the preset second criterion may be a cervical expansion item illustrated in Table 1 above.

In addition, the controller 200 may calculate a childbirth time based on the cervical angle or the degree of expansion of the cervix, determine whether a fetus is premature, and determine an abnormal childbirth state.

The controller 200 may be implemented with a memory that stores data about an algorithm for controlling the operation of components in the ultrasound diagnostic apparatus 1 or a program that reproduces the algorithm, and a processor that performs the above-described operation using data stored in the memory. In this case, the memory and the processor may be implemented as separate chips, respectively. Alternatively, the memory and the processor may be implemented as a single chip.

The display 300 may display a decision content or a decision result of the controller 200. Particularly, the display 300 may display a cervix image in which a risk of premature birth of fetus, a childbirth state, and the schematic cervical angle are displayed, or a degree of expansion of the cervix together with an expanded value.

In addition, the controller 200 may control the display 300 to determine a childbirth stage according to a change in cervical angle or a change in degree of expansion of the cervix, and control the display 300 to display an appropriate action to the user. For example, the display 300 may display a vaginal incision, the childbirth stage, or a mother's condition according to the change in cervical angle or the change in degree of expansion of the cervix.

On the other hand, the display 300 may be provided as a cathode ray tube (CRT), a digital light processing (DLP) panel, a plasma display panel, a liquid crystal display (LCD) panel, an electro Luminescence (EL) panel, an electrophoretic display (EPD) panel, an electrochromic display (ECD) panel, a light emitting diode (LED) panel, or an organic light emitting diode (OLED) panel, but is not limited thereto.

The storage 400 may store the image data obtained by the probe 100 and may store a control result of the controller 200.

The storage 400 may be implemented with at least one of a non-volatile memory device, such as a cache, Read Only Memory (ROM), Programmable ROM (PROM), Erasable Programmable ROM (EPROM), and Electrically Erasable Programmable ROM (EEPROM), a volatile memory device, such as Random Access Memory (RAM), or a storage medium, such as Hard Disk Drive (HDD) and Compact Disk (CD) ROM, without being limited thereto. The storage 400 may be the memory implemented as a chip separate from the processor associated with the controller 600, and may be implemented as the single chip with the processor.

The storage 400 may store the image data obtained by the probe 100 and store the control result of the controller 200, so that the ultrasound diagnostic apparatus 1 may be converted a childbirth situation into big data and combined with artificial intelligence to suggest a method for the user to take appropriate action when a similar situation arrives.

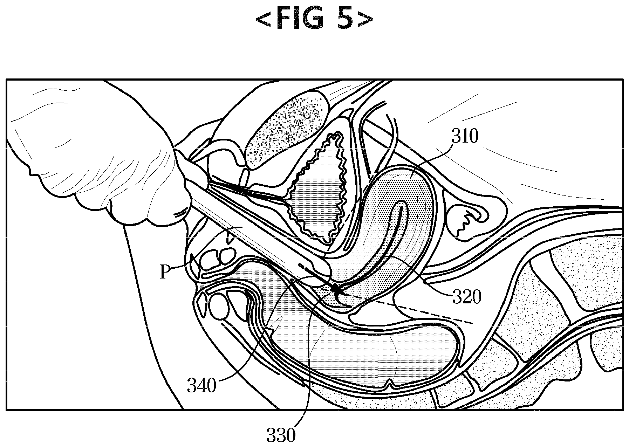

FIG. 5 is a view illustrating a process of obtaining image data of a cervix of an object according to an embodiment.

Referring to FIG. 5, the user may perform diagnosis by inserting the probe 100 into a body of the object. Particularly, the user may obtain diagnostic data on a uterus 310 of the object by inserting the probe 100 toward a cervix 330 of the object.

The controller 200 may obtain a sagittal plane or a transverse plane of the uterus 310 of the object. In addition, the controller 200 may obtain data on the cervical angle of the uterus 310 of the object or the degree of expansion of the cervix.

The sagittal plane of the uterus 310 may be obtained when the user enters an upper vaginal cavity with a marked area of the probe 100 at 12 o'clock direction.

The transverse plane of the uterus 310 may be obtained when the user rotates the marked area of the probe 100 along a long axis 340 in a counterclockwise direction from a middle sagittal plane of the uterus 310 by 90 degrees. In this case, the transverse plane of the uterus 310 may be obtained by rotating 90 degrees along the long axis 340 in a clockwise direction from the middle sagittal plane.

Further, the cervical angle and the degree of expansion of the cervix may be determined based on the image data of the anterior or posterior of the cervix.

The controller 130 may determine whether the fetus is premature, the childbirth time or the childbirth stage based on the sagittal plane, transverse plane, the cervical angle, or the degree of expansion of the cervix illustrated in the ultrasound image of the uterus of the object.

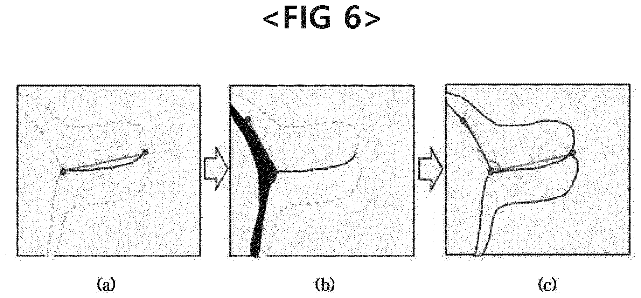

FIG. 6 is a view illustrating a cervical angle appearing on a display according to an embodiment.

Referring to FIG. 6, the controller 200 may control the display 300 to determine the cervical angle based on the image data obtained by the probe 100 and display the determined angle by synthesizing the determined angle on a uterus image.

Particularly, referring to FIG. 6A, the controller 200 may display both ends of the anterior of the cervix with a connected straight line.

In addition, referring to FIG. 6B, the controller 200 may display the anterior end point of the cervix in a direction inside the uterus 310 and a point at which the posterior starts with the connected straight line. In addition, the controller 200 may process an empty space where the fetus and the uterus are not in contact with a black shade.

Referring to FIG. 6C, the controller 200 may synthesize the images generated in FIGS. 6A and 6B, and control the display 300 to display an image in which the cervical angle is displayed. Particularly, the image in which the cervical angle is displayed may include a straight line connecting both ends of the anterior of the cervix, a straight line connecting the end of the anterior of the cervix in the direction inside the uterus and the point where the posterior starts, and the angle between the two straight lines.

Through the processes of FIGS. 6A, 6B, and 6C, the controller 200 may generate an image in which the straight line indicating the cervical angle is displayed based on the obtained image data information.

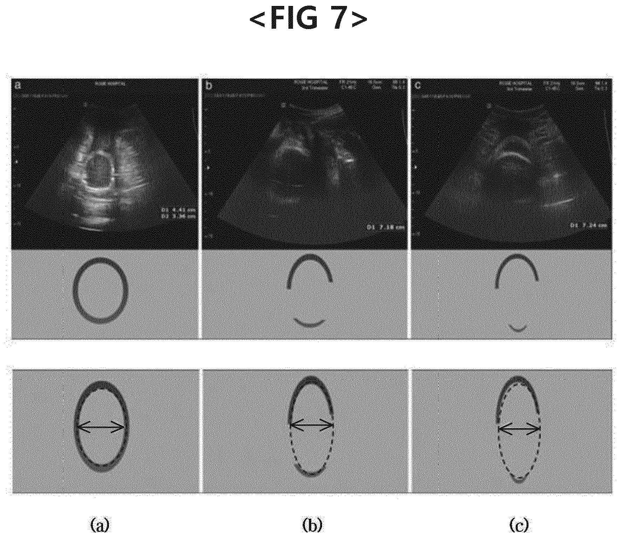

FIG. 7 is a view illustrating a degree of expansion of a cervix appearing on a display according to an embodiment.

Particularly, FIG. 7A illustrates that the probe 100 obtains all image data regarding the degree of expansion of the cervix, and determines the degree of expansion of the cervix based on the data obtained by the controller 200.

However, FIGS. 7B and 7C illustrate that when the probe 100 obtains only part of the image data regarding the degree of expansion of the cervix, the controller 200 determines the degree of expansion of the cervix by ellipse fitting and displays the expansion of the cervix on the display 300.

Through the processes of FIGS. 7A, 7B, and 7C, the controller 200 may generate an accurate image of the cervix even if all the image data of the cervix is not obtained due to factors such as noise.

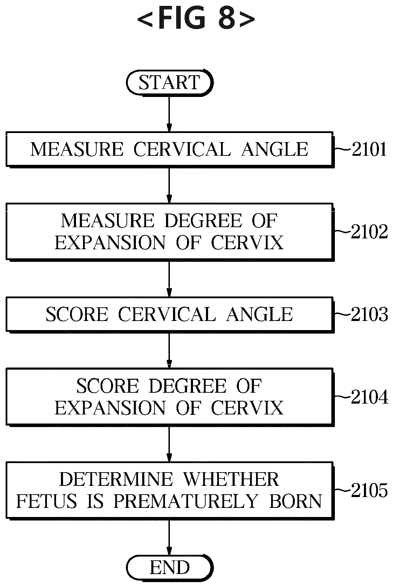

FIG. 8 is a view illustrating a process of determining whether a fetus is prematurely born by a controller according to an embodiment.

Referring to FIG. 8, the probe 100 may obtain the image data of the cervix, and the controller 200 may measure the cervical angle by analyzing the obtained image data (2101). As described above, the cervical angle may refer to an angle formed by the straight line connecting both ends of the anterior of the cervix and the straight line connecting the end of the anterior of the cervix in the direction inside the uterus 310 and the point at which the posterior starts.

Further, the probe 100 obtains the image data of the cervix, and the controller 200 measures the degree of expansion of the cervix by analyzing the obtained image data (2102).

Particularly, the degree of expansion of the cervix may be determined from an anterior image or a posterior image of the cervix. In addition, even when only part of the image data is obtained, as described above, the controller 200 controls the display 300 to display the image indicating the degree of expansion of the cervix by ellipse fitting.

Upon determining the cervical angle, the controller 200 may score the cervical angle (2103). The cervical angle may be classified as an anterior cervical angle or a posterior cervical angle according to a measured viewpoint.

The controller 200 may assign 0 point when the anterior cervical angle exceeds 141°, 1 point when the anterior cervical angle exceeds 101° and is less than 141°, and 2 points when the anterior cervical angle is less than 100°. However, a score assigned by the controller 100 is not limited thereto and may vary according to a user definition or a score calculation method.

In addition, the controller 200 may assign 0 point when the posterior cervical angle is less than 100°, 1 point when the posterior cervical angle is less than 141°, and 2 points when the posterior cervical angle exceeds 141°. However, the score assigned by the controller 100 is not limited thereto and may vary according to the user definition or the score calculation method.

In addition, when the degree of expansion of the cervix is measured, the controller 200 may score the degree of expansion of the cervix (2104). Particularly, the controller 200 may calculate the score based on an expanded width of the cervix.

The controller 200 may assign 0 point when it is determined that the cervix is not open, 1 point when it is determined that the cervix is open by 1cm to 2cm, 2 points when it is determined that the cervix is open by 3cm to 4cm, and 3 points when it is determined that the cervix is open by 5cm or more. However, the score assigned by the controller 100 is not limited thereto and may vary according to the user definition or the score calculation method.

In addition, the controller 200 may calculate the score based on factors illustrated in Table 1 in addition to the cervical angle or the degree of expansion of the cervix.

When the score based on the cervical angle, the score according to the degree of expansion of the cervix, and the score by the other factors illustrated in Table 1 are calculated, the controller 200 may determine whether fetus is prematurely born based on the calculated score ( 2105).

Particularly, when it is determined that a sum of the calculated scores is 9 or more, the controller 200 may determine a normal childbirth and do not display a separate display on the display 300. However, when it is determined that the sum of the calculated scores is less than 5 points, the controller 200 may determine an abnormal childbirth and control the display 300 to inform the user that an induction childbirth is required. In addition, when it is determined that the sum of the calculated scores is less than 3 points, the controller 300 may control the display 300 to determine that it is premature birth and that only the childbirth may fail even if the induction childbirth is performed.

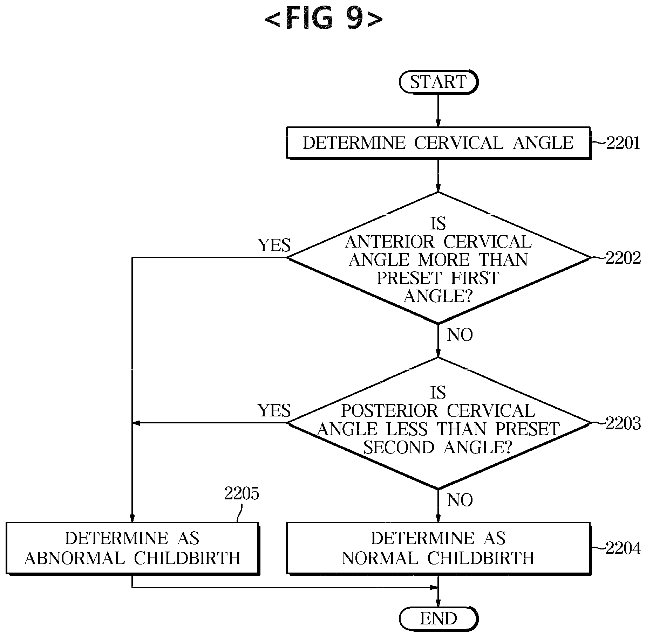

FIG. 9 is a view illustrating how a controller determines a mother's childbirth state based on a cervical angle, according to an embodiment.

Referring to FIG. 9, the controller 200 may determine the cervical angle based on the data obtained by the probe 100 (2201). As described above, the cervical angle may include the anterior cervical angle or the posterior cervical angle.

When the cervical angle is determined, the controller 200 may determine whether the anterior cervical angle exceeds a preset first angle (2202). Here, the first angle may be 141° based on the anterior cervical angle, but is not limited thereto.

When it is determined that the anterior cervical angle exceeds the first angle, the controller 200 may determine that it is the abnormal childbirth and control the display 300 to display the childbirth state.

Here, the abnormal childbirth may be a situation in which the childbirth progresses before 37 weeks of pregnancy, but is not limited thereto, and may refer to a preterm birth situation in which the childbirth progresses before a preset pregnancy order.

However, when it is determined that the anterior cervical angle is less than the first angle, the controller 200 may determine whether the posterior cervical angle is less than the preset second angle (2203). Here, the second angle may be 100° based on the posterior cervical angle, but is not limited thereto.

When it is determined that the posterior cervical angle is less than the second angle, the controller 200 may determine that it is the abnormal childbirth and control the display 300 to display the childbirth state.

However, when it is determined that the anterior cervical angle exceeds the first angle or the posterior cervical angle is less than the second angle, the controller 200 may determine that the childbirth is normal and terminate a control process (2204).

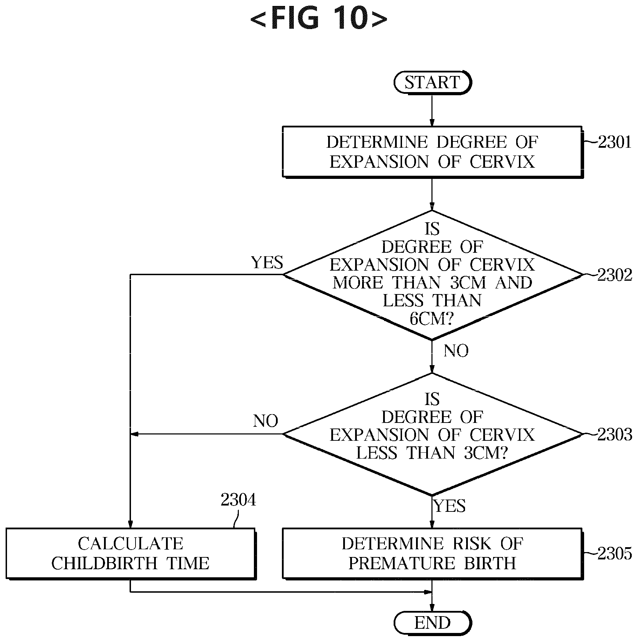

FIG. 10 is a view illustrating a process in which a controller determines a childbirth time and a risk of premature birth based on a degree of expansion of a cervix, according to an embodiment.

The controller 200 may determine the degree of expansion of the cervix based on the data obtained by the probe 100 (2301).

When the degree of expansion of the cervix is determined, the controller 200 may determine whether the degree of expansion of the cervix is 3cm or more and less than 6cm (2302).

When the degree of expansion of the cervix is determined, when the degree of expansion of the cervix is determined to be 3cm or more and less than 6cm, the controller 200 may determine that it is the normal childbirth and calculate the childbirth time (2304). However, when it is determined that the degree of expansion of the cervix is not less than 3cm and less than 6cm, the controller 200 may determine whether the degree of expansion of the cervix is less than 3cm..

When it is determined that the degree of expansion of the cervix is less than 3cm, the controller 200 may determine that there is the risk of premature birth (2305), and control the display 300 to display the risk of premature birth. However, when it is determined that the degree of expansion of the cervix is not less than 3cm, the controller 300 may determine that it is the normal childbirth and calculate the childbirth time (2304).

Here, the controller 200 may calculate a childbirth time of 48 hours when the degree of expansion of the cervix is less than 3cm, and a childbirth time within 24 hours when the degree of expansion of the cervix is 3cm or more, but is not limited thereto.

According to the ultrasound diagnostic apparatus and the method of processing the ultrasound diagnostic apparatus according to the embodiments, it is possible to determine the correct cervical angle and the degree of expansion of the cervix, and to determine the childbirth state, without a medical staff's seismic examination.

According to the ultrasound diagnostic apparatus and the method of processing the ultrasound diagnostic apparatus according to the embodiments, there is an effect of accurately identifying whether the fetus is premature or not and promoting the safety of the mother and fetus.

The disclosed embodiments may be implemented in the form of a recording medium storing computer-executable instructions that are executable by a processor. The instructions may be stored in the form of a program code, and when executed by a processor, the instructions may generate a program module to perform operations of the disclosed embodiments. The recording medium may be implemented non-transitory as a computer-readable recording medium.

The non-transitory computer-readable recording medium may include all kinds of recording media storing commands that can be interpreted by a computer. For example, the non-transitory computer-readable recording medium may be, for example, ROM, RAM, a magnetic tape, a magnetic disc, flash memory, an optical data storage device, etc.

Embodiments of the disclosure have thus far been described with reference to the accompanying drawings. It should be obvious to a person of ordinary skill in the art that the disclosure may be practiced in other forms than the embodiments as described above. The above embodiments are only by way of example, and should not be interpreted in a limited sense.

## Claims

1. An ultrasound diagnostic apparatus comprising:
a display (300);
a probe (100) configured to obtain image data of a cervix using ultrasound; and
a controller (200) configured to:
determine a degree of expansion of the cervix based on the image data, generate an ultrasound image of the cervix, control the display to output an image in which the degree of expansion of the cervix is displayed, **characterised in that** the controller is configured to determine the degree of expansion of the cervix by ellipse fitting.

2. The ultrasound diagnostic apparatus according to claim 1, wherein the controller is configured to schematically display at least one of a cervical angle and the degree of expansion of the cervix at a position corresponding to the cervix in the ultrasound image.

3. The ultrasound diagnostic apparatus according to claim 2, wherein the controller is configured to calculate a score of the cervical angle based on a first predetermined criterion, and to determine a childbirth stage based on the calculated score of the cervical angle.

4. The ultrasound diagnostic apparatus according to claim 1, wherein the controller is configured to score an expansion of the cervix based on a preset second criterion and determine a childbirth stage.

5. The ultrasound diagnostic apparatus according to claim 1, wherein the controller is configured to calculate a childbirth time based on the degree of expansion of the cervix.

6. The ultrasound diagnostic apparatus according to claim 1, wherein the controller is configured to determine whether a fetus risks to be prematurely born based on the image data, and to control the display to display the determination result on the display.

7. The ultrasound diagnostic apparatus according to claim 1, wherein the controller is configured to measure an anterior cervical angle or a posterior cervical angle, and to determine that it is an abnormal childbirth in response to the anterior cervical angle exceeding a preset first angle or the posterior cervical angle being less than a preset second angle.

8. The ultrasound diagnostic apparatus according to claim 7, wherein, in response to the anterior cervical angle being more than 101° or less than 140°, or the posterior cervical angle being more than 101° or less than 140°, the controller is configured to determine that it is the abnormal childbirth.

9. The ultrasound diagnostic apparatus according to claim 1, further comprising:
a storage (400) configured to store the image data

10. A method of controlling an ultrasound diagnostic apparatus comprising:
obtaining, by a probe, image data of a cervix using ultrasound;
determining, by a controller, a degree of expansion of the cervix based on the image data;
generating, by the controller, an ultrasound image of the cervix;
controlling, by a controller, the display to output an image in which the degree of expansion of the cervix is displayed, **characterised in that** the degree of expansion of the cervix is determined by ellipse fitting.

11. The method according to claim 10, wherein the controlling further comprises:
schematically displaying at least one of a cervical angle and the degree of expansion of the cervix at a position corresponding to the cervix in the ultrasound image.

12. The method according to claim 11, wherein the controlling further comprises:
calculating a score of the cervical angle based on a first predetermined criterion; and
determining a childbirth stage based on the calculated score of the cervical angle.

13. The method according to claim 10, wherein the controlling further comprises:
scoring an expansion of the cervix based on a preset second criterion and determine a childbirth stage.

14. The method according to claim 10, wherein the controlling further comprises:
calculating a childbirth time based on the degree of expansion of the cervix.

15. The method according to claim 10, wherein the controlling further comprises:
determining whether a fetus risks to be prematurely born based on the image data; and
controlling the display to display the determination result on the display.

## Patentansprüche

1. Ultraschalldiagnosevorrichtung, die Folgendes umfasst:
eine Anzeige (300);
eine Sonde (100), die so ausgelegt ist, dass sie unter Verwendung von Ultraschall Bilddaten eines Gebärmutterhalses erhält; und
eine Steuerung (200), die zu Folgendem ausgelegt ist:
Bestimmen eines Ausdehnungsgrades des Gebärmutterhalses basierend auf den Bilddaten,
Erzeugen eines Ultraschallbildes des Gebärmutterhalses, und
Steuern der Anzeige zur Ausgabe eines Bildes, in dem der Ausdehnungsgrad des Gebärmutterhalses angezeigt wird,
**dadurch gekennzeichnet, dass** die Steuerung so ausgelegt ist, dass sie das Bestimmen des Ausdehnungsgrades des Gebärmutterhalses durch Ellipsenanpassung vornimmt.

2. Ultraschalldiagnosevorrichtung nach Anspruch 1, wobei die Steuerung so ausgelegt ist, dass sie einen Gebärmutterhalswinkel und/oder den Ausdehnungsgrad des Gebärmutterhalses an einer dem Gebärmutterhals im Ultraschallbild entsprechenden Position schematisch anzeigt.

3. Ultraschalldiagnosevorrichtung nach Anspruch 2, wobei die Steuerung so ausgelegt ist, dass sie eine Bewertung des Gebärmutterhalswinkels basierend auf einem ersten vorgegebenen Kriterium berechnet und ein Geburtsstadium basierend auf der berechneten Bewertung des Gebärmutterhalswinkels bestimmt.

4. Ultraschalldiagnosevorrichtung nach Anspruch 1, wobei die Steuerung so ausgelegt ist, dass sie eine Ausdehnung des Gebärmutterhalses basierend auf einem voreingestellten zweiten Kriterium bewertet und ein Geburtsstadium bestimmt.

5. Ultraschalldiagnosevorrichtung nach Anspruch 1, wobei die Steuerung so ausgelegt ist, dass sie basierend auf dem Ausdehnungsgrad des Gebärmutterhalses einen Geburtszeitpunkt berechnet.

6. Ultraschalldiagnosevorrichtung nach Anspruch 1, wobei die Steuerung so ausgelegt ist, dass sie basierend auf den Bilddaten bestimmt, ob für einen Fötus das Risiko einer Frühgeburt besteht, und dass sie die Anzeige steuert, um das Ergebnis der Bestimmung auf der Anzeige anzuzeigen.

7. Ultraschalldiagnosevorrichtung nach Anspruch 1, wobei die Steuerung so ausgelegt ist, dass sie einen anterioren Gebärmutterhalswinkel oder einen posterioren Gebärmutterhalswinkel misst und als Reaktion darauf, dass der anteriore Gebärmutterhalswinkel einen voreingestellten ersten Winkel übersteigt oder der posteriore Gebärmutterhalswinkel kleiner als ein voreingestellter zweiter Winkel ist, bestimmt, dass es sich um eine anormale Geburt handelt.

8. Ultraschalldiagnosevorrichtung nach Anspruch 7, wobei die Steuerung so ausgelegt ist, dass sie als Reaktion darauf, dass der anteriore Gebärmutterhalswinkel größer als 101° oder kleiner als 140° oder der posteriore Gebärmutterhalswinkel größer als 101° oder kleiner als 140° ist, bestimmt, dass es sich um eine anormale Geburt handelt.

9. Ultraschalldiagnosevorrichtung nach Anspruch 1, die ferner Folgendes umfasst:
einen Speicher (400), der so ausgelegt ist, dass er die Bilddaten speichert.

10. Verfahren zum Steuern einer Ultraschalldiagnosevorrichtung, wobei das Verfahren Folgendes umfasst:
Erhalten von Bilddaten eines Gebärmutterhalses durch eine Sonde unter Verwendung von Ultraschall;
Bestimmen eines Ausdehnungsgrades des Gebärmutterhalses basierend auf den Bilddaten durch eine Steuerung;
Erzeugen eines Ultraschallbildes des Gebärmutterhalses durch die Steuerung; und
Steuern einer Anzeige zur Ausgabe eines Bildes, in dem der Ausdehnungsgrad des Gebärmutterhalses angezeigt wird, durch die Steuerung;
**dadurch gekennzeichnet, dass** der Ausdehnungsgrad des Gebärmutterhalses durch Ellipsenanpassung bestimmt wird.

11. Verfahren nach Anspruch 10, wobei das Steuern ferner Folgendes umfasst:
schematisches Anzeigen eines Gebärmutterhalswinkels und/oder des Ausdehnungsgrades des Gebärmutterhalses an einer dem Gebärmutterhals im Ultraschallbild entsprechenden Position.

12. Verfahren nach Anspruch 11, wobei das Steuern ferner Folgendes umfasst:
Berechnen einer Bewertung des Gebärmutterhalswinkels basierend auf einem ersten vorbestimmten Kriterium; und
Bestimmen eines Geburtsstadiums basierend auf der berechneten Bewertung des Gebärmutterhalswinkels.

13. Verfahren nach Anspruch 10, wobei das Steuern ferner Folgendes umfasst:
Bewerten einer Ausdehnung des Gebärmutterhalses basierend auf einem voreingestellten zweiten Kriterium und Bestimmen eines Geburtsstadiums.

14. Verfahren nach Anspruch 10, wobei das Steuern ferner Folgendes umfasst:
Berechnen eines Geburtszeitpunkts basierend auf dem Ausdehnungsgrad des Gebärmutterhalses.

15. Verfahren nach Anspruch 10, wobei das Steuern ferner Folgendes umfasst:
Bestimmen, ob für einen Fötus das Risiko einer Frühgeburt besteht, basierend auf den Bilddaten; und
Steuern der Anzeige, um das Ergebnis der Bestimmung auf der Anzeige anzuzeigen.

## Revendications

1. Appareil d'échographie diagnostique comprenant :
un dispositif d'affichage (300),
une sonde (100) conçue pour obtenir des données d'image d'un col de l'utérus à l'aide d'ultrasons, et
un organe de commande (200) conçu pour :
déterminer un degré de dilatation du col compte tenu des données d'image,
générer une image échographique du col de l'utérus, et
commander le dispositif d'affichage de manière qu'il produise une image dans laquelle est affiché le degré de dilatation du col ;
**caractérisé en ce que** l'organe de commande est conçu pour déterminer le degré de dilatation du col par régression elliptique.

2. Appareil d'échographie diagnostique selon la revendication 1, dans lequel l'organe de commande est conçu pour afficher schématiquement un angle cervical et/ou le degré de dilatation du col en un endroit correspondant au col dans l'image échographique.

3. Appareil d'échographie diagnostique selon la revendication 2, dans lequel l'organe de commande est conçu pour calculer un score de l'angle cervical compte tenu d'un premier critère prédéterminé, et pour déterminer le stade de l'accouchement compte tenu du score de l'angle cervical calculé.

4. Appareil d'échographie diagnostique selon la revendication 1, dans lequel l'organe de commande est conçu pour évaluer la dilatation du col compte tenu d'un deuxième critère prédéfini et déterminer le stade de l'accouchement.

5. Appareil d'échographie diagnostique selon la revendication 1, dans lequel l'organe de commande est conçu pour calculer l'heure d'accouchement compte tenu du degré de dilatation du col.

6. Appareil d'échographie diagnostique selon la revendication 1, dans lequel l'organe de commande est conçu pour déterminer si un foetus risque de naître prématurément compte tenu des données d'image, et pour commander le dispositif d'affichage de manière qu'il affiche le résultat de la détermination.

7. Appareil d'échographie diagnostique selon la revendication 1, dans lequel l'organe de commande est conçu pour mesurer un angle cervical antérieur ou un angle cervical postérieur, et pour déterminer qu'il s'agit d'un accouchement anormal si l'angle cervical antérieur dépasse un premier angle prédéfini ou si l'angle cervical postérieur est inférieur à un deuxième angle prédéfini.

8. Appareil d'échographie diagnostique selon la revendication 7, dans lequel, lorsque l'angle cervical antérieur est supérieur à 101° ou inférieur à 140°, ou que l'angle cervical postérieur est supérieur à 101° ou inférieur à 140°, l'organe de commande est conçu pour déterminer qu'il s'agit d'un accouchement anormal.

9. Appareil d'échographie diagnostique selon la revendication 1, comprenant en outre :
un dispositif de stockage (400) conçu pour stocker les données d'image.

10. Procédé de commande d'un appareil d'échographie diagnostique comprenant :
l'obtention, au moyen d'une sonde, de données d'image d'un col de l'utérus à l'aide d'ultrasons,
la détermination, par un organe de commande, du degré de dilatation du col compte tenu des données d'image,
la génération, par l'organe de commande, d'une image échographique du col de l'utérus, et
la commande, par l'organe de commande, d'un dispositif d'affichage de manière qu'il produise une image dans laquelle est affiché le degré de dilatation du col ;
**caractérisé en ce que** le degré de dilatation du col est déterminé par régression elliptique.

11. Procédé selon la revendication 10, dans lequel la commande comprend en outre :
l'affichage schématique d'un angle cervical et/ou du degré de dilatation du col en un endroit correspondant au col dans l'image échographique.

12. Procédé selon la revendication 11, dans lequel la commande comprend en outre :
le calcul d'un score de l'angle cervical compte tenu d'un premier critère prédéterminé, et
la détermination du stade de l'accouchement compte tenu du score de l'angle cervical calculé.

13. Procédé selon la revendication 10, dans lequel la commande comprend en outre :
l'évaluation de la dilatation du col de l'utérus compte tenu d'un deuxième critère prédéfini et la détermination du stade de l'accouchement.

14. Procédé selon la revendication 10, dans lequel la commande comprend en outre :
le calcul de l'heure d'accouchement en fonction du degré de dilatation du col.

15. Procédé selon la revendication 10, dans lequel la commande comprend en outre :
la détermination de fait ou non qu'un foetus risque de naître prématurément compte tenu des données d'image, et
la commande du dispositif d'affichage de manière qu'il affiche le résultat de la détermination.
